# EUROPÄISCHE PATENTSCHRIFT

(11) **EP 1 534 711 B1**
(45) Veröffentlichungstag und Bekanntmachung des Hinweises auf die Patenterteilung: **19.04.2006**
(21) Anmeldenummer: 03792301.8
(22) Anmeldetag: 12.08.2003
(51) Int. Cl.: C07D 487/04, A61K 31/519, A61P 25/00, C07D 239/00, C07D 231/00

(54) **PHENYL-SUBSTITUIERTE PYRAZOLOPYRIMIDINE**
PHENYL-SUBSTITUTED PYRAZOLOPYRIMIDINES
PYRAZOLOPYRIMIDINES SUBSTITUEES PAR PHENYLE

(30) Priorität: 23.08.2002 DE 10238723
(43) Veröffentlichungstag der Anmeldung: 01.06.2005
(73) Patentinhaber: Bayer HealthCare AG, 51368 Leverkusen (DE)
(72) Erfinder: HENDRIX, Martin, 51519 Odenthal (DE); BÖSS, Frank-Gerhard, Berkshire SL5 7DF (GB); BURKHARDT, Nils, 42553 Velbert (DE); ERB, Christina, 65830 Kriftel (DE); TERSTEEGEN, Adrian, 42553 Velbert (DE); VAN KAMPEN, Marja, D-40223 Dusseldorf (DE)
(86) Internationale Anmeldenummer: PCT/EP2003/008923
(87) Internationale Veröffentlichungsnummer: WO 2004/018474

(56) Entgegenhaltungen:
- WO-A-98/40384
- DE-B- 1 147 234
- DE-B- 1 149 013
- GB-A- 937 726

## Beschreibung

Die Erfindung betrifft neue Phenyl-substituierte Pyrazolopyrimidine, Verfahren zu ihrer Herstellung, und ihre Verwendung zur Herstellung von Arzneimitteln zur Verbesserung von Wahrnehmung, Konzentrationsleistung, Lern- und/oder Gedächtnisleistung.

Die zelluläre Aktivierung von Adenylat- bzw. Guanylatzyklasen bewirkt die Zyklisierung von ATP bzw. GTP zu 5'-3' zyklischem Adenosin-Monophosphat (cAMP) bzw. 5'-3' zyklischem Guanosin-Monophosphat (cGMP). Diese zyklischen Nukleotide (cAMP und cGMP) sind wichtige second messenger und spielen daher eine zentrale Rolle in den zellulären Signaltransduktionskaskaden. Beide aktivieren unter anderem, aber nicht ausschließlich, jeweils wieder Protein-Kinasen. Die von cAMP aktivierte Protein-Kinase wird Protein-Kinase A (PKA) genannt, die von cGMP aktivierte Protein-Kinase wird Protein-Kinase G (PKG) genannt. Aktivierte PKA bzw. PKG können wiederum eine Reihe zellulärer Effektorproteine phosphorylieren (z.B. Ionenkanäle, G-Protein gekoppelte Rezeptoren, Strukturproteine). Auf diese Weise können die second messengers cAMP und cGMP die unterschiedlichsten physiologischen Vorgänge in den verschiedensten Organen kontrollieren. Die zyklischen Nukleotide können aber auch direkt auf Effektormoleküle wirken. So ist z.B. bekannt, dass cGMP direkt auf Ionenkanäle wirken kann und hiermit die zelluläre Ionenkonzentration beeinflussen kann (Übersicht in: Wei et al., *Prog. Neurobiol.,* **1998,** *56*: 37-64). Ein Kontrollmechanismus, um die Aktivität von cAMP und cGMP und damit diese physiologischen Vorgänge wiederum zu steuern, sind die Phosphodiesterasen (PDE). PDEs hydrolysieren die zyklischen Monophosphate zu den inaktiven Monophosphaten AMP und GMP. Es sind mittlerweile mindestens 21 PDE-Gene beschrieben (*Exp. Opin. Investig. Drugs* **2000,** *9*, 1354-3784). Diese 21 PDE-Gene lassen sich aufgrund ihrer Sequenzhomologie in 11 PDE-Familien einteilen (Nomenklatur Vorschlag siehe http://depts.washington.edu/pde/Nomenclature.html.). Einzelne PDE-Gene innerhalb einer Familie werden durch Buchstaben unterschieden (z.B. PDE1A und PDEIB). Falls noch unterschiedliche Splice Varianten innerhalb eines Genes vorkommen, wird dies dann durch eine zusätzliche Nummerierung nach dem Buchstaben angegeben (z.B. PDE1A1).

Die humane PDE9A wurde 1998 kloniert und sequenziert. Die Aminosäurenidentität zu anderen PDEs liegt bei maximal 34 % (PDE8A) und minimal 28 % (PDE5A). Mit einer Michaelis-Menten-Konstante (Km-Wert) von 170 nM ist PDE9A hochaffin für cGMP. Darüber hinaus ist PDE9A selektiv für cGMP (Km-Wert für cAMP = 230 µM). PDE9A weist keine cGMP Bindungsdomäne auf, die auf eine allosterische Enzymregulation durch cGMP schließen ließe. In einer Westem-Blot-Analyse wurde gezeigt, daß die PDE9A im Mensch in Hoden, Gehirn, Dünndarm, Skelettmuskulatur, Herz, Lunge, Thymus und Milz exprimiert wird. Die höchste Expression wurde in Gehirn, Dünndarm, Herz und Milz gefunden (Fisher et al., *J*. *Biol. Chem.,* **1998**, *273* (25): 15559-15564). Das Gen für die humane PDE9A liegt auf Chromosom 21q22.3 und enthält 21 Exons. Bislang wurden 4 alternative Spleißvarianten der PDE9A identifiziert (Guipponi et al., *Hum. Genet.,* **1998**, *103:* 386-392). Klassische PDE-Inhibitoren hemmen die humane PDE9A nicht. So zeigen IBMX, Dipyridamole, SKF94120, Rolipram und Vinpocetin in Konzentrationen bis 100 µM keine Inhibition am isolierten Enzym. Für Zaprinast wurde ein IC₅₀-Wert von 35 µM nachgewiesen (Fisher et al., *J. Biol. Chem.,* **1998,** *273* (25): 15559-15564).

Die Maus-PDE9A wurde 1998 von Soderling et al. *(J. Biol. Chem.,* **1998,** *273* (19): 15553-15558) kloniert und sequenziert. Diese ist wie die humane Form hochaffin für cGMP mit einem Km von 70 nM. In der Maus wurde eine besonders hohe Expression in der Niere, Gehirn, Lunge und Herz gefunden. Auch die Maus-PDE9A wird von IBMX in Konzentrationen unter 200 µM nicht gehemmt; der IC₅₀-Wert für Zaprinast liegt bei 29 µM (Soderling et al., *J*. *Biol. Chem.,* **1998**, *273* (19): 15553-15558). Im Rattengehirn wurde gezeigt, daß PDE9A in einigen Hirnregionen stark exprimiert wird. Dazu zählen der Bulbus olfactorius, Hippocampus, Cortex, Basalganglien und basales Vorderhirn (Andreeva et al., *J*. *Neurosci.,* **2001**, *21* (22): 9068-9076). Insbesondere Hippokampus, Cortex und basales Vorderhirn spielen eine wichtige Rolle an Lern- und Gedächtnisvorgängen.

Wie oben bereits erwähnt, zeichnet sich PDE9A durch eine besonders hohe Affinität für cGMP aus. Deshalb ist PDE9A im Gegensatz zu PDE2A (Km = 10 µM; Martins et al., *J. Biol. Chem.,* **1982**, 257: 1973-1979), PDE5A (Km = 4 µM; Francis et al., *J. Biol. Chem.,* **1980,** *255:* 620-626), PDE6A (Km = 17 µM; Gillespie and Beavo, *J. Biol. Chem.,* **1988***, 263* (17): 8133-8141) und PDE11A (Km = 0.52 µM; Fawcett et al., *Proc. Nat. Acad. Sci.,* **2000,** 97 (7): 3702-3707) schon bei niedrigen physiologischen Konzentrationen aktiv. Im Gegensatz zu PDE2A (Murashima et al., *Biochemistry,* **1990**, *29*: 5285-5292) wird die katalytische Aktvität von PDE9A nicht durch cGMP gesteigert, da es keine GAF-Domäne (cGMP-Bindedomäne, über die die PDE-Aktivität allosterisch gesteigert wird) aufweist (Beavo et al., *Current Opinion in Cell Biology,* **2000,** *12*: 174-179). PDE9A-Inhibitoren führen deshalb zu einer Erhöhung der basalen cGMP-Konzentration. Diese Erhöhung der basalen cGMP-Konzentration führte überraschenderweise zu einer Verbesserung der Lern-und Gedächtnisleistung im Social Recognition Test.

Die WO 98/40384 offenbart Pyrazolopyrimidine, die sich als PDE1-, 2- und 5-Inhibitoren auszeichnen und für die Behandlung von cardiovascularen, cerebrovascularen Erkrankungen sowie Erkrankungen des Urogenitalbereiches eingesetzt werden können.

In CH 396 924, CH 396 925, CH 396 926, CH 396 927, DE 1 147 234, DE 1 149 013, GB 937,726 werden Pyrazolopyrimidine mit coronarerweiternder Wirkung beschrieben, die zur Behandlung von Durchblutungsstörungen des Herzmuskels eingesetzt werden können.

Im US 3,732,225 werden Pyrazolopyrimidine beschrieben, die eine entzündungshemmende und Blutzucker-senkende Wirkung haben.

In DE 2 408 906 werden Styrolpyrazolopyrimidine beschrieben, die als antimikrobielle und entzündungshemmende Mittel für die Behandlung von beispielsweise Ödem eingesetzt werden können.

Die vorliegende Erfindung betrifft Verbindungen der Formel 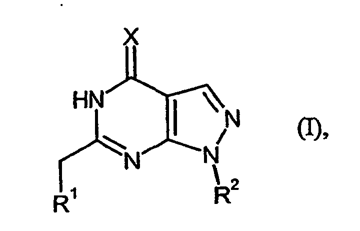 in welcher
- R¹: Phenyl, welches mit 1 bis 5 Substituenten unabhängig voneinander ausgewählt aus der Gruppe Halogen, C₁-C₆-Alkyl, Trifluormethyl, Trifluormethoxy, Cyano, Hydroxy, Nitro und C₁-C₆-Alkoxy substituert ist,
- R²: Pentan-3-yl, C₄-C₆-Cycloalkyl,
- X: Sauerstoff oder Schwefel,
bedeuten,
sowie deren Salze, Solvate und/oder Solvate der Salze.

Erfindungsgemäße Verbindungen sind die Verbindungen der Formel (I) und deren Salze, Solvate und Solvate der Salze; die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Als Salze sind im Rahmen der Erfindung physiologisch unbedenkliche Salze der erfindungsgemäßen Verbindungen bevorzugt.

Physiologisch unbedenkliche Salze der Verbindungen (I) umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure und Benzoesäure.

Physiologisch unbedenkliche Salze der Verbindungen (I) umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Dehy droabietylamin, Arginin, Lysin, Ethylendiamin und Methylpiperidin.

Als Solvate werden im Rahmen der Erfindung solche Formen der Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem umfasst die vorliegende Erfindung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
C₁-C₆-Alkoxy steht für einen geradkettigen oder verzweigten Alkoxyrest mit 1 bis 6, bevorzugt 1 bis 4, besonders bevorzugt mit 1 bis 3 Kohlenstoffatomen. Bevorzugte Beispiele sind Methoxy, Ethoxy, n-Propoxy, Isopropoxy, tert.-Butoxy, n-Pentoxy und n-Hexoxy.
C₁-C₆-Alkyl steht für einen geradkettigen oder verzweigten Alkylrest mit 1 bis 6, bevorzugt 1 bis 4, besonders bevorzugt 1 bis 3 Kohlenstoffatomen. Bevorzugte Beispiele sind Methyl, Ethyl, n-Propyl, Isopropyl, tert.-Butyl, n-Pentyl und n-Hexyl.
C₄-C₆- und C₅-C₆-Cycloalkyl stehen für gesättigte oder teilweise ungesättigte Cycloalkylreste mit 4 bis 6, bevorzugt 5 bis 6 Kohlenstoffatomen. Bevorzugte Beispiele sind Cyclobutyl, Cyclopentyl und Cyclohexyl.
Halogen steht für Fluor, Chlor, Brom und Iod. Bevorzugt sind Fluor, Chlor, Brom, besonders bevorzugt Fluor und Chlor.

Wenn Reste in den erfindungsgemäßen Verbindungen gegebenenfalls substituiert sind, ist, soweit nicht anders spezifiziert, eine Substitution mit bis zu drei gleichen oder verschiedenen Substituenten bevorzugt.

Die erfindungsgemäßen Verbindungen können auch als Tautomere vorliegen, wie im Folgenden beispielhaft gezeigt wird:

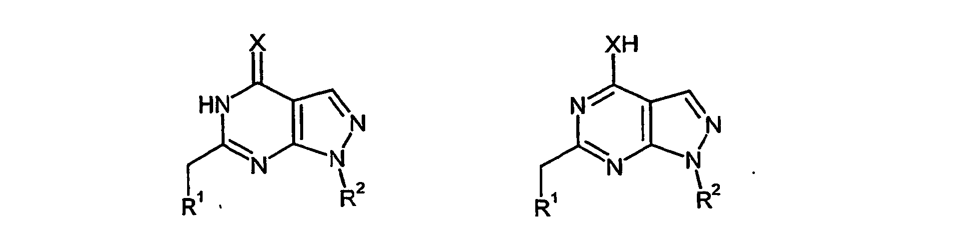

Eine weitere Ausführungsform der Erfindung betrifft Verbindungen der Formel (I), in welcher
- R¹: Phenyl, welches mit 1 bis 3 Substituenten unabhängig voneinander ausgewählt aus der Gruppe Fluor, Chlor, Brom, C₁-C₄-Alkyl, Trifluormethyl, Trifluormethoxy, Cyano, Hydroxy, Nitro und C₁-C₄-Alkoxy substituert ist,
- R²: Pentan-3-yl, C₅-C₆-Cycloalkyl,
- X: Sauerstoff oder Schwefel,
bedeuten, sowie deren Salze, Solvate und/oder Solvate der Salze.

Eine weitere Ausführungsform der Erfindung betrifft Verbindungen der Formel 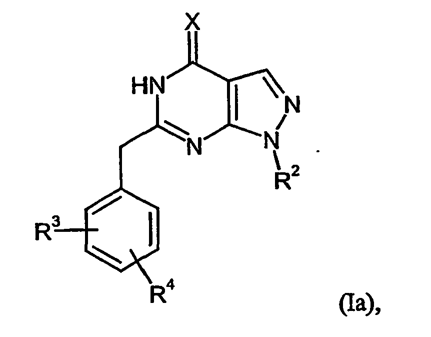 in welcher
- R³: Wasserstoff oder Chlor,
- R⁴: Fluor, Chlor, Brom, Methyl, Trifluormethyl,
- R²: Pentan-3-yl, Cyclopentyl,
- X: Sauerstoff oder Schwefel,
bedeuten, sowie deren Salze, Solvate und/oder Solvate der Salze.

Eine weitere Ausführungsform der Erfindung betrifft Verbindungen der Formeln (I) und (Ia),
in welcher
- R³: Wasserstoff oder Chlor,
- R⁴: Fluor, Chlor, Brom, Methyl, Trifluormethyl,
- R²: Pentan-3-yl, Cyclopentyl,
- X: Sauerstoff,
bedeuten, sowie deren Salze, Solvate und/oder Solvate der Salze.

Außerdem wurde ein Verfahren zur Herstellung der erfindungsgemäßen Verbindungen gefunden, dadurch gekennzeichnet, dass man entweder
[A] Verbindungen der Formel 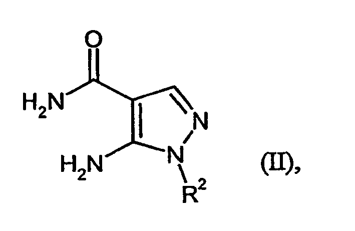 in welcher
   - R²: die oben angegebenen Bedeutungen hat,
   durch Umsetzung mit einer Verbindung der Formel

   R¹-CH₂-C(O)-Z (IIIa),

   in welcher
   - R¹: die oben angegebenen Bedeutungen hat
   und
   - Z: für Chlor oder Brom steht,
   in einem inerten Lösemittel und in Anwesenheit einer Base zunächst in Verbindungen der Formel 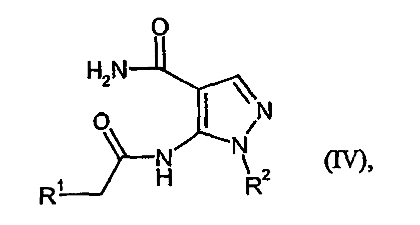 in welcher
   R¹ und R² die oben angegebenen Bedeutungen haben,
   überführt, dann in einem inerten Lösemittel in Gegenwart einer Base zu Verbindungen der Formel 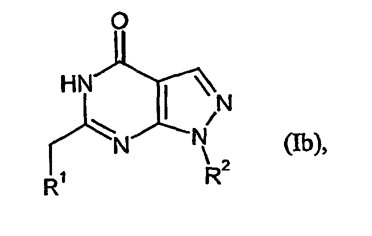 in welcher
   R¹ und R² die oben angegebenen Bedeutungen haben,
   cyclisiert,
   oder
[B] Verbindungen der Formel (II) unter direkter Cyclisierung zu (Ib) mit einer Verbindung der Formel

   R¹-CH₂-C(O)-OR⁵ (IIIb),

   in welcher
   - R¹: die oben angegebenen Bedeutungen hat
   und
   - R⁵: für Methyl oder Ethyl steht,
   in einem inerten Lösemittel und in Anwesenheit einer Base umsetzt,
   oder
[C] Verbindungen der Formel 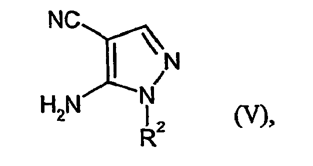 in welcher
   - R²: die oben angegebenen Bedeutungen hat,
   zunächst durch Umsetzung mit einer Verbindung der Formel (IIIa) in einem inerten Lösemittel und in Anwesenheit einer Base in Verbindungen der Formel 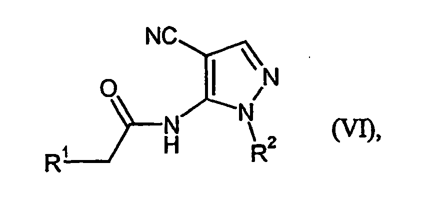 in welcher
   R¹ und R² die oben angegebenen Bedeutungen haben,
   überführt,
   und diese in einem zweiten Schritt in einem inerten Lösemittel und in Anwesenheit einer Base und eines Oxidationsmittels zu (Ib) cyclisiert,
   und die Verbindungen der Formel (Ib) gegebenenfalls dann durch Umsetzung mit einem Schwefelungsmittel wie beispielsweise Diphosphorpentasulfid in die Thiono-Derivate der Formel 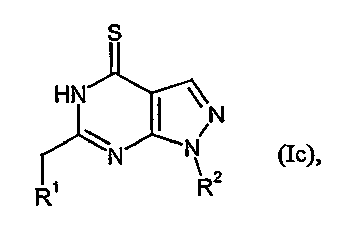 in welcher
   R¹ und R² die oben angegebenen Bedeutungen haben,
   überführt,
   und die resultierenden Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

Für den ersten Schritt des Verfahrens [A] und des Verfahrens [C] eignen sich inerte organische Lösemittel, die sich unter den Reaktionsbedingungen nicht verändern. Hierzu gehören bevorzugt Ether wie beispielsweise Diethylether, Dioxan, Tetrahydrofuran oder Glykoldimethylether, oder Toluol oder Pyridin. Ebenso ist es möglich, Gemische der genannten Lösemittel einzusetzen. Besonders bevorzugt sind Tetrahydrofuran, Toluol oder Pyridin.

Als Basen eignen sich im allgemeinen Alkalihydride, wie beispielsweise Natriumhydrid, oder cyclische Amine, wie beispielsweise Piperidin, Pyridin, Dimethylaminopyridin (DMAP), oder C₁-C₄-Alkylamine, wie beispielsweise Triethylamin. Bevorzugt sind Natriumhydrid, Pyridin und/oder Dimethylaminopyridin.

Die Base wird im allgemeinen in einer Menge von 1 mol bis 4 mol, bevorzugt von 1.2 mol bis 3 mol, jeweils bezogen auf 1 mol der Verbindungen der Formel (II) bzw. (V), eingesetzt.

In einer Variante wird die Umsetzung in Pyridin, dem eine katalytische Menge DMAP zugesetzt wird, durchgeführt. Gegebenenfalls kann noch Toluol zugefügt werden.

Die Reaktionstemperatur kann im allgemeinen in einem größeren Bereich variiert werden. Im allgemeinen arbeitet man in einem Bereich von -20°C bis +200°C, bevorzugt von 0°C bis +100°C.

Als Lösemittel für die Cyclisierung im zweiten Schritt der Verfahren [A] und [C] eignen sich die üblichen organischen Lösemittel. Hierzu gehören bevorzugt Alkohole wie Methanol, Ethanol, Propanol, Isopropanol, n-Butanol oder tert.-Butanol, oder Ether wie Tetrahydrofuran oder Dioxan, oder Dimethylformamid oder Dimethylsulfoxid. Besonders bevorzugt werden Alkohole wie Methanol, Ethanol, Propanol, Isopropanol oder tert.-Butanol verwendet. Ebenso ist es möglich, Gemische der genannten Lösemittel einzusetzen.

Als Basen für die Cyclisierung im zweiten Schritt der Verfahren [A] und [C] eignen sich die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.-butanolat. Besonders bevorzugt sind Kaliumcarbonat, Natriumhydroxid und Kalium-tert.-butanolat.

Bei der Durchführung der Cyclisierung wird die Base im allgemeinen in einer Menge von 2 mol bis 6 mol, bevorzugt von 3 mol bis 5 mol, jeweils bezogen auf 1 mol der Verbindungen der Formel (IV) bzw. (VI), eingesetzt.

Als Oxidationsmittel für die Cyclisierung im zweiten Schritt des Verfahrens [C] eignen sich beispielsweise Wasserstoffperoxid oder Natriumborat. Bevorzugt ist Wasserstoffperoxid.

Die Cyclisierung in den Verfahren [A], [B] und [C] wird im allgemeinen in einem Temperaturbereich von 0°C bis +160°C, bevorzugt bei der Siedetemperatur des jeweiligen Lösemittels durchgeführt.

Die Cyclisierung wird im allgemeinen bei Normaldruck durchgeführt. Es ist aber auch möglich, das Verfahren bei Überdruck oder bei Unterdruck durchzuführen (z.B. in einem Bereich von 0.5 bis 5 bar).

Als Lösemittel für das Verfahren [B] eignen sich die oben für den zweiten Schritt der Verfahren [A] und [C] aufgeführten Alkohole, wobei Ethanol bevorzugt ist.

Als Basen für das Verfahren [B] eignen sich Alkalihydride, wie beispielsweise Natrium- oder Kaliumhydrid, oder Alkalialkoholate, wie beispielsweise Natriummethanolat, -ethanolat, -isopropylat oder Kalium-tert.-butylat. Bevorzugt ist Natriumhydrid.

Die Base wird in einer Menge von 2 mol bis 8 mol, bevorzugt von 3 mol bis 6 mol, jeweils bezogen auf 1 mol der Verbindungen der Formel (II), eingesetzt

Die Verbindungen der Formel (II) sind bekannt oder können beispielsweise hergestellt werden, indem man zunächst Ethoxymethylenmalonsäuredinitril mit Hydrazin-Derivaten der Formel

R²-NH-NH₂ (VII),

in welcher
R² die oben angegebenen Bedeutungen hat,
in einem inerten Lösemittel zu den Pyrazolnitrilen der Formel (V) kondensiert und diese dann mit einem der oben aufgeführten Oxidationsmittel, vorzugsweise Wasserstoffperoxid, in Anwesenheit von Ammoniak umsetzt [vgl. z.B. A. Miyashita et al., Heterocycles 1990, 31, 1309ff].

Die Verbindungen der Formeln (IIIa), (IIIb) und (VII) sind kommerziell erhältlich, literaturbekannt oder können in Analogie zu literaturbekannten Verfahren hergestellt werden.

Das erfindungsgemäße Verfahren kann durch das folgendes Formelschema beispielhaft erläutert werden:

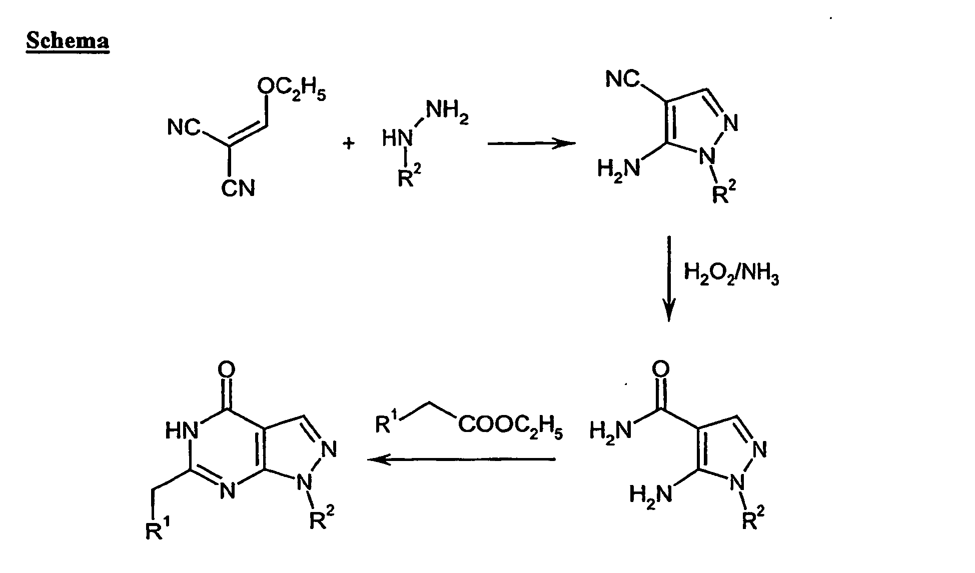

Weitere Verfahren zur Herstellung von Pyrazolo[3,4-d]pyrimidin-4-onen sind bekannt und können ebenfalls zur Synthese der erfindungsgemäßen Verbindungen eingesetzt werden (siehe zum Beispiel: P. Schmidt et al., *Helvetica Chimica Acta* **1962**, *189*, 1620ff.).

Die erfindungsgemäßen Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren.

Der Begriff "Behandlung" im Rahmen der vorliegenden Erfindung schließt die Prophylaxe ein.

Überraschenderweise wurde gefunden, dass selektive PDE9A-Inhibitoren zur Herstellung von Arzneimitteln zur Verbesserung der Wahrnehmung, Konzentrationsleistung, Lernleistung oder Gedächtnisleistung geeignet sind.

Die erfindungsgemäßen Verbindungen können aufgrund ihrer pharmakologischen Eigenschaften allein oder in Kombination mit anderen Arzneimitteln zur Verbesserung von Wahrnehmung, Konzentrationsleistung, Lern- und/oder Gedächtnisleistung eingesetzt werden.

Ein PDE9A-Inhibitor im Sinne der Erfindung ist eine Verbindung, die humane PDE9A unter den unten angegebenen Bedingungen mit einem IC₅₀-Wert von weniger als 10 µM, bevorzugt weniger als 1 µM hemmt.

Ein selektiver PDE9A-Inhibitor im Sinne der Erfindung ist eine Verbindung, die humane PDE9A unter den unten angegebenen Bedingungen stärker hemmt als die humanen PDE1C, PDE2A, PDE3B, PDE4B, PDE5A, PDE7B, PDE8A, PDE10A und PDE11. Bevorzugt ist ein Verhältnis von IC₅₀ (PDE9A) / IC₅₀ (PDE1C, PDE2A, PDE3B, PDE4B, PDE5A, PDE7B und PDE10A) kleiner als 0.2.

Besonders eignen sich die selektiven PDE9A-Inhibitoren zur Verbesserung der Wahrnehmung, Konzentrationsleistung, Lernleistung, oder Gedächtnisleistung nach Kognitiven Störungen, wie sie insbesondere bei Situationen/Krankheiten/Syndromen auftreten wie "Mild cognitive impairment", Altersassoziierte Lern- und Gedächtnisstörungen, Altersassoziierte Gedächtnisverluste, Vaskuläre Demenz, Schädel-Hirn-Trauma, Schlaganfall, Demenz, die nach Schlaganfällen auftritt ("post stroke dementia"), post-traumatische Demenz, allgemeine Konzentrationsstörungen, Konzentrationsstörungen in Kindern mit Lern- und Gedächtnisproblemen, Alzheimer'sche Krankheit, Demenz mit Lewy-Körperchen, Demenz mit Degeneration der Frontallappen einschließlich des Pick's Syndroms, Parkinson'sche Krankheit, Progressive nuclear palsy, Demenz mit corticobasaler Degeneration, Amyotrope Lateralsklerose (ALS), Huntingtonsche Krankheit, Multiple Sklerose, Thalamische Degeneration, Creutzfeld-Jacob-Demenz, HIV-Demenz, Schizophrenie mit Demenz oder Korsakoff-Psychose.

Die in vitro-Wirkung der erfindungsgemäßen Verbindungen kann mit folgenden biologischen Assays gezeigt werden:

### PDE-Inhibition

Rekombinante PDE1C (GenBank/EMBL Accession Number: NM_005020, Loughney et al. *J*. *Biol. Chem.* **1996** *271*, 796-806), PDE2A (GenBank/EMBL Accession Number: NM_002599, Rosman et al. Gene **1997** *191,* 89-95), PDE3B (GenBank/EMBL Accession Number: NM_000922, Miki et al. *Genomics* **1996***, 36,* 476-485), PDE4B (GenBank/EMBL Accession Number: NM_002600, Obernolte et al. *Gene.* **1993**, *129,* 239-247), PDE5A (GenBank/EMBL Accession Number: NM_001083, Loughney et al. *Gene* **1998**, *216*, 139-147), PDE7B (GenBank/EMBL Accession Number: NM_018945, Hetman et al. *Proc. Natl. Acad. Sci. U.S.A.* **2000***,* 97, 472-476), PDE8A (GenBank/EMBL Accession Number: AF_056490, Fisher et al. *Biochem. Biophys. Res. Commun.* **1998** 246, 570-577), PDE9A (Fisher et al., J. Biol. Chem, 1998, 273 (25): 15559-15564), PDE10A (GenBank/EMBL Accession Number: NM_06661, Fujishige et al. *J Biol Chem.* **1999*****,** 274,* 18438-45.), PDE11A (GenBank/EMBL Accession Number: NM_016953, Fawcett et al. *Proc. Natl. Acad. Sci.* **2000**, *97*, 3702-3707) wurden mit Hilfe des pFASTBAC Baculovirus Expressionssystems (GibcoBRL) in Sf9-Zellen exprimiert.

Die Testsubstanzen werden zur Bestimmung ihrer *in vitro* Wirkung an PDE 9A in 100 % DMSO aufgelöst und seriell verdünnt. Typischerweise werden Verdünnungsreihen von 200 µM bis 1.6 µM hergestellt (resultierende Endkonzentrationen im Test: 4 µM bis 0.032 µM). Jeweils 2 µL der verdünnten Substanzlösungen werden in die Vertiefungen von Mikrotiterplatten (Isoplate; Wallac Inc., Atlanta, GA) vorgelegt. Anschließend werden 50 µL einer Verdünnung des oben beschriebenen PDE9A-Präparates hinzugefiigt. Die Verdünnung des PDE9A-Präparates wird so gewählt, dass während der späteren Inkubation weniger als 70 % des Substrates umgesetzt wird (typische Verdünnung: 1: 10000; Verdünnungspuffer: 50 mM Tris/HCl pH 7.5, 8.3 mM MgCl₂, 1.7 mM EDTA, 0.2% BSA). Das Substrat, [8-³H] guanosine 3',5'-cyclic phosphate (1 µCi/µL; Amersham Pharmacia Biotech., Piscataway, NJ) wird 1:2000 mit Assaypuffer (50 mM Tris/HCl pH 7.5, 8.3 mM MgCl₂, 1.7 mM EDTA) auf eine Konzentration von 0.0005 µCi/µL verdünnt. Durch Zugabe von 50 µL (0.025 µCi) des verdünnten Substrates wird die Enzymreaktion schließlich gestartet. Die Testansätze werden für 60 min bei Raumtemperatur inkubiert und die Reaktion durch Zugabe von 25 µl eines in Assaypuffer gelösten PDE9A-Inhibitors (z.B. der Inhibitor aus Herstellbeispiel 1,10 µM Endkonzentration) gestoppt. Direkt im Anschluß werden 25 µL einer Suspension mit 18 mg/mL Yttrium Scintillation Proximity Beads (Amersham Pharmacia Biotech., Piscataway, NJ) hinzugefügt. Die Mikrotiterplatten werden mit einer Folie versiegelt und für 60 min bei Raumtemperatur stehengelassen. Anschließend werden die Platten für 30 s pro Vertiefung in einem Microbeta Szintillationzähler (Wallac Inc., Atlanta, GA) vermessen. IC₅₀₋Werte werden anhand der graphischen Auftragung der Substanzkonzentration gegen die prozentuale Inhibition bestimmt.

Die *in vitro* Wirkung von Testsubstanzen an rekombinanter PDE3B, PDE4B, PDE7B, PDE8A, PDE10A und PDE11A wird nach dem oben für PDE 9A beschriebenen Testprotokoll mit folgenden Anpassungen bestimmt: Als Substrat wird [5',8-³H] adenosine 3',5'-cyclic phosphate (1 µCi/µL; Amersham Pharmacia Biotech., Piscataway, NJ) verwendet. Die Zugabe einer Inhibitorlösung zum Stoppen der Reaktion ist nicht notwendig. Stattdessen wird in Anschluß an die Inkubation von Substrat und PDE direkt mit der Zugabe der Yttrium Scintillation Proximity Beads wie oben beschrieben fortgefahren und dadurch die Reaktion gestoppt. Für die Bestimmung einer entsprechenden Wirkung an rekombinanter PDE1C, PDE2A und PDE5A wird das Protokoll zusätzlich wie folgt angepaßt: Bei PDE1C werden zusätzlich Calmodulin 10⁻⁷ M und CaCl₂ 3 mM zum Reaktionsansatz gegeben. PDE2A wird im Test durch Zugabe von cGMP 1 µM stimuliert und mit einer BSA-Konzentration von 0.01 % getestet. Für PDE1C und PDE2A wird als Substrat [5',8-³H] adenosine 3',5'-cyclic phosphate (1 µCi/µL; Amersham Pharmacia Biotech., Piscataway, NJ), für PDE5A [8-³H] guanosine 3',5'-cyclic phosphate (1 µCi/µL; Amersham Pharmacia Biotech., Piscataway, NJ) eingesetzt.

Die PDE9A-inhibierende Wirkung der erfindungsgemäßen Verbindungen kann anhand der folgenden Beispiele gezeigt werden:

**Tabelle 1:**

| Beispiel | IC₅₀ [nM] |
|---|---|
| 1 | 20 |
| 2 | 30 |
| 4 | 30 |
| 10 | 64 |
| 13 | 30 |

### Erhöhung der intrazellulären neuronalen cGMP-Konzentration in Zellkulturen

PDE9A-Inhibitoren erhöhen die intrazelluläre neuronale cGMP in kultivierten primären kortikalen Neuronen.

Rattenembryonen (Embryonaltag E17- E19) wurden dekapitiert, die Köpfe in mit Präparationsmedium (DMEM, Penicillin/Streptomycin; beides von Gibco) gefüllte Präparationsschalen überführt. Die Kopfhaut und Schädeldecke wurde entfernt, und die freipräparierten Gehirne wurden in eine weitere Petrischale mit Präparationsmedium überführt. Mithilfe eines Binokulars und zweier Pinzetten wurde das Großhirn (Kortex) isoliert und mit Eis auf 4°C gekühlt. Diese Präparation und die Vereinzelung der kortikalen Neuronen wurden dann nach einem Standardprotokoll mit dem Papain-Kit (Worthington Biochemical Corporation, Lakewood, New Jersey 08701, USA) durchgeführt (Huettner et al. *J. Neurosci.* **1986,** *6*, 3044-3060). Die mechanisch vereinzelten kortikalen Neurone wurden zu 150.000 Zellen/Loch in 200 µl Neurobasahnedium/Loch (Neurobasal; B27 Supplement; 2 mM L-Glutamin; in Anwesenheit von Penicillin/Streptomycin; alle Agenzien von Gibco) 7 Tage in 96 Lochplatten (mit Poly-D-Lysin 100 µg/ml für 30 min vorbehandelt) unter Standard-Bedingungen kultiviert (37°C, 5 % CO₂). Nach 7 Tagen wurde das Medium abgenommen und die Zellen mit HBSS-Puffer (Hank's balanced salt solution, Gibco/BRL) gewaschen. Anschließend werden 100 µl erfindungsgemäße Verbindung in HBSS-Puffer gelöst (zuvor in 100 % DMSO gelöst: 10 mM) auf die Zellen gegeben. Anschließend werden nochmals 100 µl HBSS-Puffer zugegeben, sodass die Endkonzentration der erfindungsgemäßen Verbindungen beispielsweise in einem Bereich von 20 nM bis 10 µM liegt, und bei 37°C für 20 min inkubiert. Der Testpuffer wird danach komplett abgenommen. Anschließend werden die Zellen in 200 µl Lysispuffer (cGMP Kit code RPN 226; von Amersham Pharmacia Biotech.) lysiert und die cGMP-Konzentration nach den Angaben des Herstellers gemessen. Alle Messungen werden in Triplikaten durchgeführt. Die statistische Auswertung erfolgt mit Prism Software Version 2.0 (GraphPad Software Inc., San Diego, CA USA).

Inkubation der primären Neuronen mit den erfindungsgemäßen Verbindungen führte zu einer Steigerung des cGMP-Gehaltes.

### Langzeitpotenzierung

Langzeitpotenzierung wird als ein zelluläres Korrelat für Lern- und Gedächtnisvorgänge angesehen. Zur Bestimmung, ob PDE9-Inhibition einen Einfluß auf Langzeitpotenzierung hat, kann folgende Methode angewandt werden:

Rattenhippokampi werden in einen Winkel von etwa 70 Grad im Verhältnis zur Schnittklinge plaziert (Chopper). In Abständen von 400 µm wird der Hippokampus zerschnitten. Die Schnitte werden mit Hilfe eines sehr weichen, stark benetzten Pinsels (Marderhaar) von der Klinge genommen und in ein Glasgefäß mit carbogenisierter gekühlter Nährlösung (124 mM NaCl, 4.9 mM KCl, 1.3 mM MgSO₄ x 7 H₂O, 2.5 mM CaCl₂ wasserfrei, 1.2 mM KH₂PO₄, 25.6 mM NaHCO₃, 10 mM Glucose, pH 7.4) überführt. Während der Messung befinden sich die Schnitte in einer temperierten Kammer unter einem Flüssigkeitsspiegel von 1-3 mm Höhe. Die Durchflußrate beträgt 2.5 ml/min. Die Vorbegasung erfolgt unter geringen Überdruck (etwa 1 atm) sowie über eine Mikrokanüle in der Vorkammer. Die Schnittkammer ist mit der Vorkammer so verbunden, dass eine Minizirkulation aufrechterhalten werden kann. Als Antrieb der Minizirkulation wird das durch die Mikrokanüle ausströmende Carbogen eingesetzt. Die frisch präparierten Hippokampusschnitte werden mindestens 1 Stunde bei 33°C in der Schnittkammer adaptiert.

Die Reizstärke wird so gewählt, dass die fokalen exzitatiorischen postsynaptischen Potentiale (fEPSP) 30 % des maximalen exzitatorischen postsynaptischen Potentials (EPSP) betragen. Mit Hilfe einer monopolaren Stimulationselektrode, die aus lackiertem Edelstahl besteht, und eines stromkonstanten, biphasischen Reizgenerators (AM-Systems 2100) werden lokal die Schaffer-Kollateralen erregt (Spannung: 1-5 V, Impulsbreite einer Polarität 0.1 ms, Gesamtimpuls 0.2 ms). Mit Hilfe von Glaselektroden (Borosilikatglas mit Filament, 1-5 MOhm, Durchmesser: 1.5 mm, Spitzendurchmesser: 3-20 µm), die mit normaler Nährlösung gefüllt sind, werden aus dem Stratum radiatum die exzitatorischen postsynaptischen Potentiale (fEPSP) registriert. Die Messung der Feldpotentiale geschieht gegenüber einer chlorierten Referenzelektrode aus Silber, die sich am Rande der Schnittkammer befindet, mit Hilfe eines Gleichspannungsverstärkers. Das Filtern der Feldpotentiale erfolgt über einen Low-Pass Filter (5 kHz). Für die statistische Analyse der Experimente wird der Anstieg (slope) der fEPSPs (fEPSP-Anstieg) ermittelt. Die Aufnahme, Analyse und Steuerung des Experimentes erfolgt mit Hilfe eines Softwareprogrammes (PWIN), welches in der Abteilung Neurophysiologie entwickelt worden ist. Die Mittelwertbildung der fEPSP-Anstiegswerte zu den jeweiligen Zeitpunkten und die Konstruktion der Diagramme erfolgt mit Hilfe der Software EXCEL, wobei ein entsprechendes Makro die Aufnahme der Daten automatisiert.

Superfusion der Hippokampusschnitte mit einer 10 µM Lösung der erfindungsgemäßen Verbindungen führt zu einer signifikanten Steigerung der LTP.

### Sozialer Wiedererkennungstest:

Der Soziale Wiedererkennungstest ist ein Lern- und Gedächtnistest. Er misst die Fähigkeit von Ratten, zwischen bekannten und unbekannten Artgenossen zu unterscheiden. Deshalb eignet sich dieser Test zur Prüfung der lern- oder gedächtnisverbessernden Wirkung der erfindungsgemäßen Verbindungen.

Adulte Ratten, die in Gruppen gehalten werden, werden 30 min vor Testbeginn einzeln in Testkäfige gesetzt. Vier min vor Testbeginn wird das Testtier in eine Beobachtungsbox gebracht. Nach dieser Adaptationszeit wird ein juveniles Tier zu dem Testtier gesetzt und 2 min lang die absolute Zeit gemessen, die das adulte Tier das Junge inspiziert (Trial 1). Gemessen werden alle deutlich auf das Jungtier gerichteten Verhaltensweisen, d.h. ano-genitale Inspektion, Verfolgen sowie Fellpflege, bei denen das Alttier einen Abstand von höchstens 1 cm zu dem Jungtier hatte. Danach wird das Juvenile herausgenommen, das Adulte mit einer erfindungsgemäßen Verbindung oder Vehikel behandelt und anschließend in seinen Heimkäfig zurückgesetzt Nach einer Retentionszeit von 24 Stunden wird der Test wiederholt (Trial 2). Eine verringerte Soziale Interaktionszeit im Vergleich zu Trial 1 zeigt an, daß die adulte Ratte sich an das Jungtier erinnert.

Die adulten Tiere werden direkt im Anschluß an Trial 1 entweder mit Vehikel (10 % Ethanol, 20 % Solutol, 70 % physiologische Kochsalzlösung) oder 0.1 mg/kg, 0.3 mg/kg, 1.0 mg/kg bzw. 3.0 mg/kg erfindungsgemäßer Verbindung, gelöst in 10 % Ethanol, 20 % Solutol, 70 % physiologische Kochsalzlösung intraperitoneal injiziert. Vehikel behandelte Ratten zeigen keine Reduktion der sozialen Interaktionszeit in Trial 2 verglichen mit Trial 1. Sie haben folglich vergessen, dass sie schon einmal Kontakt mit dem Jungtier hatten. Überraschenderweise ist die soziale Interaktionszeit im zweiten Durchgang nach Behandlung mit den erfindungsgemäßen Verbindungen signifikant gegenüber den Vehikel behandelten reduziert. Dies bedeutet, daß die substanzbehandelten Ratten sich an das juvenile Tier erinnert haben und somit die erfindungsgemäßen Verbindungen eine verbessernde Wirkung auf Lernen und Gedächtnis aufweist.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer wirksamen Menge der erfindungsgemäßen Verbindungen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart-oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im allgemeinen hat es sich als vorteilhaft erwiesen, bei parenteraler Applikation pro Tag Mengen von etwa 0.001 bis 10 mg/kg Körpergewicht zur Erzielung wirksamer Ergebnisse zu verabreichen. Bei oraler Applikation beträgt die Menge pro Tag etwa 0.005 bis 3 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### Verwendete Abkürzungen:

- DCI: direkte chemische Ionisation (bei MS)
- DCM: Dichlormethan
- DMSO: Dimethylsulfoxid
- d.Th.: der Theorie (bei Ausbeute)
- equiv.: Äquivalent(e)
- ESI: Elektrospray-Ionisation (bei MS)
- HPLC: Hochdruck-, Hochleistungsflüssigchromatographie
- MS: Massenspektroskopie
- NMR: Kernresonanzspektroskopie
- Smp.: Schmelzpunkt
- TRIS: 2-Amino-2-(hydroxymethyl)-1,3-propandiol

### Ausgangsverbindungen:

### Beispiel 1A

### 5-Amino-1-cyclohexyl-1H-pyrazol-4-carbonitril

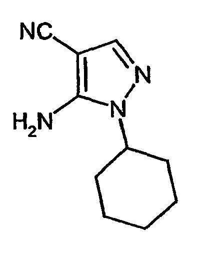

Eine Lösung von Cyclohexylhydrazin-Hydrochlorid (3 g, 19.9 mmol) in 36 ml Ethanol wird bei Raumtemperatur zunächst mit Ethoxymethylenmalonsäuredinitril (2.43 g, 19.9 mmol) und anschließend mit 8 ml Triethylamin versetzt. Das Gemisch wird 20 min refluxiert und dann abgekühlt. Das Lösungsmittel wird am Rotationsverdampfer abgezogen und der Rückstand in DCM aufgenommen, mit wässriger Natriumhydrogencarbonat-Lösung gewaschen, über Natriumsulfat getrocknet, filtriert und im Vakuum eingeengt. Das Rohprodukt wird an Kieselgel chromatographiert (Laufmittel: Dichlormethan/Methanol 0-10 %).
Ausbeute: 1.95 g (51 1 % d.Th.)
MS (DCI): m/z =191 (M+H)⁺
¹H-NMR (200 MHz, DMSO-d₆): δ = 7.5 (s, 1H), 6.5 (s, 2H), 4.0 (m, 1H), 1.95-1.05 (m, 10H) ppm.

### Beispiel 2A

### 5-Amino-1-cyclopentyl-1H-pyrazol-4-carbonitril

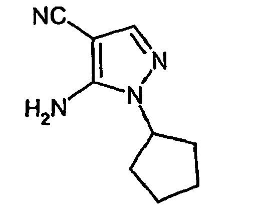

Die Herstellung erfolgt analog der Vorschrift für Beispiel 1A.
MS (ESI): m/z =177 (M+H)⁺
¹H-NMR (200 MHz, CDCl₃): δ = 7.5 (s, 1H), 4.45 (br. s, 2H), 4.35 (m, 1H), 2.2-1.55 (m, 6H) ppm.

### Beispiel 3A

### 5-Amino-1-(1-ethylpropyl)-1H-pyrazol-4-carbonitril

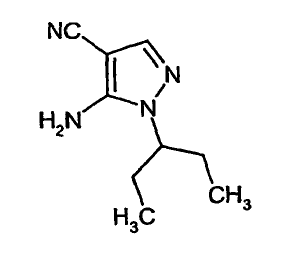

Die Herstellung erfolgt analog der Vorschrift für Beispiel 1A.
MS (ESI): m/z = 179 (M+H)⁺
¹H-NMR (300 MHz, DMSO-d₆): δ = 7.55 (s, 1H), 6.45 (s, 2H), 4.0 (m, 1H), 1.8-1.55 (m, 4H), 0.65 (t, 6H) ppm.

### Beispiel 4A

### 5-Amino-1-cyclohexyl-1H-pyrazol-4-carboxamid

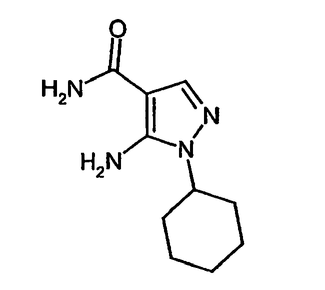

Eine Lösung von 5-Amino-1-cyclohexyl-1H-pyrazol-4-carbonitril (1.86 g, 9.81 mmol) in einem Gemisch aus 73 ml Ethanol und 90 ml konzentrierter wässriger Ammoniaklösung wird bei Raumtemperatur mit 18 ml 30%-iger Wasserstoffperoxidlösung versetzt und 1 h bei Raumtemperatur gerührt. Anschließend werden am Rotationsverdampfer die nichtwässrigen Lösemittel abgezogen. Aus der verbleibenden Mischung fällt das Produkt als Feststoff aus, der abgesaugt, mit wenig Wasser gewaschen und im Hochvakuum getrocknet wird.
Ausbeute: 1.77 g (86 % d.Th.)
MS (DCI): m/z = 209 (M+H)⁺
¹H-NMR (300 MHz, DMSO-d₆): δ = 7.6 (s, 1H), 7.3-6.4 (breit, 2H), 6.1 (s, 2H), 3.95 (m, 1H), 1.95-1.05 (m, 10H) ppm.

### Beispiel 5A

### 5-Amino-1-cyclopentyl-1H-pyrazol-4-carboxamid

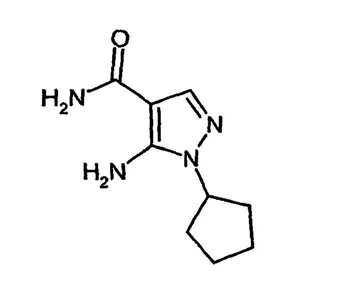

Die Herstellung erfolgt analog der Vorschrift für Beispiel 4A.
MS (ESI): m/z =195 (M+H)⁺
¹H-NMR (200 MHz, CDCl₃): δ = 7.5 (s, 1H), 5.6-4.8 (breit, 4H), 4.35 (m, 1H), 2.2-1.55 (m, 8H) ppm.

### Beispiel 6A

### 5-Amino-1-(1-ethylpropyl)-1H-pyrazol-4-carboxamid

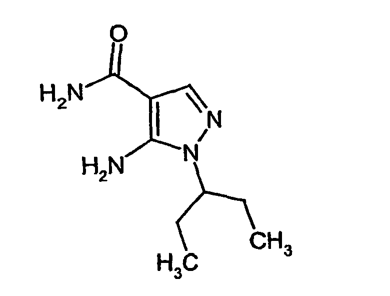

Die Herstellung erfolgt analog der Vorschrift für Beispiel 4A.
MS (ESI): m/z =197 (M+H)⁺
¹H-NMR (300 MHz, DMSO-d₆): δ = 7.65 (s, 1H), 6.9 (br. s, 2H), 6.1 (s, 2H), 3.9 (m, 1H), 1.85-1.6 (m, 4H), 0.7 (t, 6H) ppm.

### Ausführungsbeispiele:

### Beispiel 1

### 6-(3-Chlorbenzyl)-1-cyclopentyl-1,5-dihydro-4H-pyrazolo[3,4-d]pyrimidin-4-on

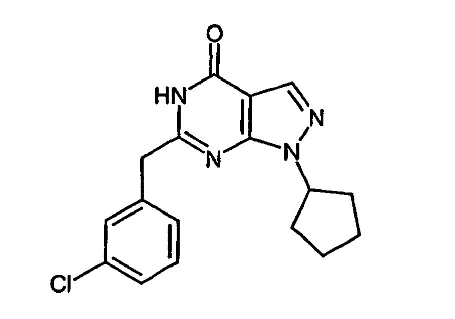

Unter Argon werden 180 mg (0.91 mmol) 5-Amino-1-cyclopentyl-1H-pyrazol-4-carboxamid und 575 mg (2.72 mmol; 3 equiv.) (3-Chlorphenyl)essigsäureethylester in 3.5 ml absolutem Ethanol vorgelegt. Bei 0°C werden 127 mg Natriumhydrid (60%-ige Dispersion in Mineralöl; 3.18 mmol; 3.5 equiv.) im Argon-Gegenstrom langsam zugegeben. Das entstandene Gemisch wird langsam erwärmt und für 18 h unter Rückfluß gerührt. Zur Aufarbeitung wird 50 ml Wasser zugegeben und das Gemisch mehrmals mit Essigsäureethylester extrahiert. Die vereinigten organischen Phasen werden über Natriumsulfat getrocknet und im Vakuum eingeengt. Das Rohprodukt wird mittels präparativer HPLC gereinigt.
Ausbeute: 244 mg (81 % d.Th.)
MS (ESI): m/z = 329 (M+H)⁺
Smp.: 159°C
¹H-NMR (200 MHz, DMSO-d₆): δ = 12.3 (s, 1H), 8.0 (s, 1H), 7.5-7.2 (m, 4H), 5.05 (m, 1H), 3.95 (s, 2H), 2.2-1.5 (m, 8H) ppm.

### Beispiel 2

### 6-(2-Fluorbenzyl)-1-cyclopentyl-1,5-dihydio-4H-pyrazolo[3,4-d]pyrimidin-4-on

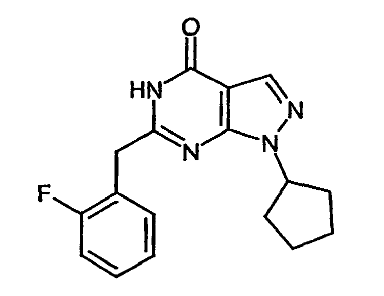

Analog zu Beispiel 1 wird das Produkt ausgehend von 100 mg (0.5 mmol) 5-Amino-1-cyclopentyl-1H-pyrazol-4-carboxamid und 260 mg (1.51 mmol) (2-Fluorphenyl)-essigsäuremethylester erhalten.
Ausbeute: 100 mg (63 % d.Th.)
MS (DCI): m/z = 313 (M+H)⁺
Smp.: 180°C
¹H-NMR (400 MHz, DMSO-d₆): δ = 12.25 (s, 1H), 8.0 (s, 1H), 7.4-7.3 (m, 2H), 7.2-7.1 (m, 2H), 4.95 (m, 1H), 4.05 (s, 2H), 2.05-1.55 (m, 8H) ppm.

### Beispiel 3

### 6-(3-Brombenzyl)-1-cyclopentyl-1,5-dihydro-4H-pyrazolo[3,4-d]pyrimidin-4-on

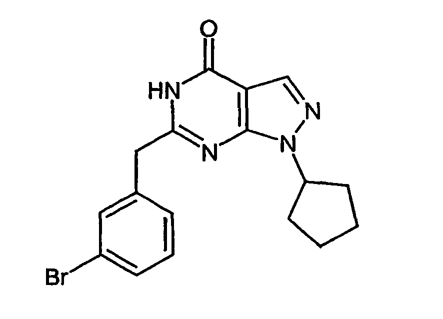

Analog zu Beispiel 1 wird das Produkt ausgehend von 80 mg (0.4 mmol) 5-Amino-1-cyclopentyl-1H-pyrazol-4-carboxamid und 277 mg (1.21 mmol) (3-Bromphenyl)-essigsäuremethylester erhalten.
Ausbeute: 93 mg (62 % d.Th.)
MS (ESI): m/z = 373 (M+H)⁺
Smp.: 159°C
¹H-NMR (400 MHz, DMSO-d₆): δ = 12.2 (s, 1H), 8.0 (s, 1H), 7.6 (s, 1H), 7.5-7.35 (m, 3H), 5.05 (m, 1H), 4.0 (s, 2H), 2.1-1.6 (m, 8H) ppm.

### Beispiel 4

### 6-(3,4-Dichlorbenzyl)-1-cyclopentyl-1,5-dihydro-4H-pyrazolo[3,4-d]pyrimidin-4-on

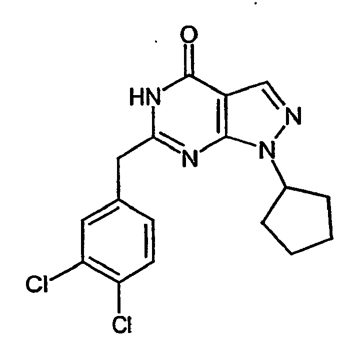

Analog zu Beispiel 1 wird das Produkt ausgehend von 75 mg (0.38 mmol) 5-Amino-1-cyclopentyl-1H-pyrazol-4-carboxamid und 254 mg (1.14 mmol) (3,4-Dichlorphenyl)essigsäuremethylester erhalten.
Ausbeute: 94 mg (68 % d.Th.)
MS (ESI): m/z = 363 (M+H)⁺
Smp.: 198°C
¹H-NMR (400 MHz, DMSO-d₆): δ =12.2 (s, 1H), 8.0 (s, 1H), 7.65 (d, 1H, *J=* 1 Hz), 7.55 (d, 1H, *J* = 7.5 Hz), 7.3 (dd, 1H, *J =* 7.5 Hz, 1 Hz), 5.05 (m, 1H), 4.0 (s, 2H), 2.1-1.6 (m, 8H) ppm.

### Beispiel 5

### 6-(3,5-Dichlorbenzyl)-1-cyclopentyl-1,5-dihydro-4H-pyrazolo[3,4-d]pyrimidin-4-on

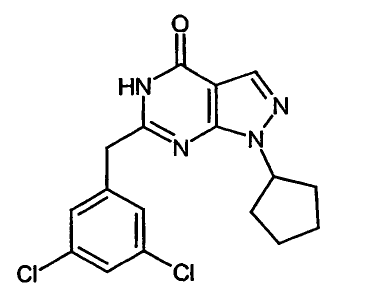

Analog zu Beispiel 1 wird das Produkt ausgehend von 150 mg (0.76 mmol) 5-Amino-1-cyclopentyl-1H-pyrazol-4-carboxamid und 507 mg (2.27 mmol) (3,5-Dichlorphenyl)essigsäuremethylester erhalten.
Ausbeute: 159 mg (58 % d.Th.)
MS (ESI): m/z = 363 (M+H)⁺
Smp.: 177°C
¹H-NMR (200 MHz, DMSO-d₆): δ = 12.25 (s, 1H), 8.0 (s, 1H), 7.55 (t, 1H, *J* = 1 Hz), 7.45 (d, 2H, *J =* Hz), 5.05 (m, 1H), 4.0 (s, 2H), 2.2-1.5 (m, 8H) ppm.

### Beispiel 6

### 6-(2,3-Dichlorbenzyl)-1-cyclopentyl-1,5-dihydro-4H-pyrazolo[3,4-d]pyrimidin-4-on

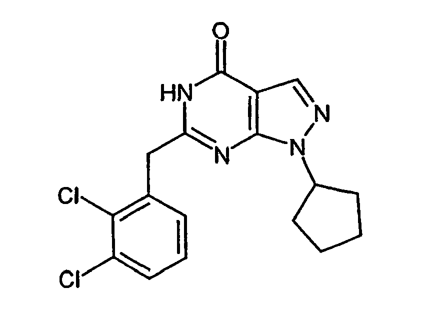

Analog zu Beispiel 1 wird das Produkt ausgehend von 150 mg (0.76 mmol) 5-Amino-1-cyclopentyl-1H-pyrazol-4-carboxamid und 406 mg (1.82 mmol) (2,3-Dichlorphenyl)essigsäuremethylester erhalten.
Ausbeute: 114 mg (41 % d.Th.)
MS (ESI): m/z = 363 (M+H)⁺
Smp.: 181°C
¹H-NMR (200 MHz, DMSO-d₆): δ = 12.35 (s, 1H), 8.0 (s, 1H), 7.6 (m, 1H), 7.4-7.3 (m, 2H), 4.9 (m, 1H), 4.2 (s, 2H), 2.1-1.5 (m, 8H) ppm.

### Beispiel 7

### 6-(3-Chlorbenzyl)-1-(1-ethylpropyl)-1,5-dihydro-4H-pyrazolo[3,4-d]pyrimidin-4-on

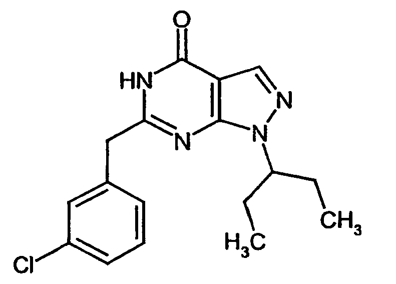

Analog zu Beispiel 1 wird das Produkt ausgehend von 150 mg (0.76 mmol) 5-Amino-1-(1-ethylpropyl)-1H-pyrazol-4-carboxamid und 484 mg (2.29 mmol) (3-Chlorphenyl)essigsäureethylester erhalten.
Ausbeute: 210 mg (83 % d.Th.)
MS (ESI): m/z = 331 (M+H)⁺
Smp.: 138°C
¹H-NMR (200 MHz, DMSO-d₆): δ = 12.3 (s, 1H), 8.0 (s, 1H), 7.45-7.25 (m, 4H), 4.45 (m, 1H), 4.0 (s, 2H), 2.0-1.7 (m, 4H), 0.6 (t, 6H, *J* = 7.5 Hz) ppm.

### Beispiel 8

### 6-(3-Methylbenzyl)-1-cyclopentyl-1,5-dffiydro-4H-pyrazolo[3,4-d]pyrimidin4-on

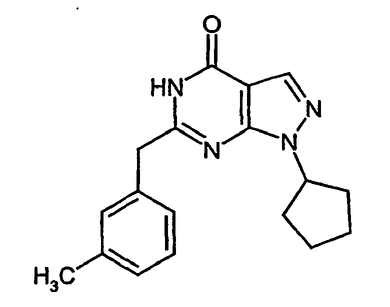

Analog zu Beispiel 1 wird das Produkt ausgehend von 200 mg (1.01 mmol) 5-Amino-1-cyclopentyl-1H-pyrazol-4-carboxamid und 550 mg (3.03 mmol) (3-Methylphenyl)essigsäureethylester erhalten.
Ausbeute: 222 mg (71 % d.Th.)
MS (ESI): m/z = 309 (M+H)⁺
Smp.: 152°C
¹H-NMR (200 MHz, DMSO-d₆): δ = 12.2 (s, 1H), 8.0 (s, 1H), 7.3-7.0 (m, 4H), 5.1 (m, 1H), 3.95 (s, 2H), 2.3 (s, 3H), 2.2-1.55 (m, 8H) ppm.

### Beispiel 9

### 6-(2,5-Dichlorbenzyl)-1-(1-ethylpropyl)-1,5-dihydro-4H-pyrazolo[3,4-d]pyrimidin-4-on

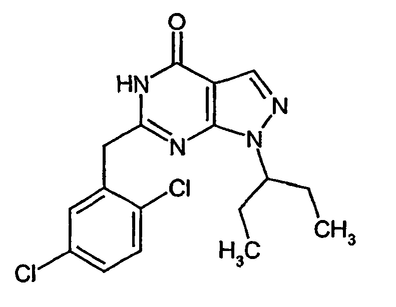

Analog zu Beispiel 1 wird das Produkt ausgehend von 200 mg (1.0 mmol) 5-Amino-1-(1-ethylpropyl)-1H-pyrazol-4-carboxamid und 806 mg (3.5 mmol) (2,5-Dichlorphenyl)essigsäuremethylester erhalten.
Ausbeute: 51 mg (14 % d.Th.)
MS (ESI): m/z = 365 (M+H)⁺
Smp.: 134°C
¹H-NMR (300 MHz, DMSO-d₆): δ =12.3 (s, 1H), 8.0 (s, 1H), 7.55-7.35 (m, 3H), 4.2 (m, 1H), 4.15 (s, 2H), 1.9-1.65 (m, 4H), 0.55 (t, 6H, *J =* 7.5 Hz) ppm.

### Beispiel 10

### 6-(3-Methylbenzyl)-1-(1-ethylpropyl)-1,5-dihydro-4H-pyrazolo[3,4-d]pyrimidin-4-on

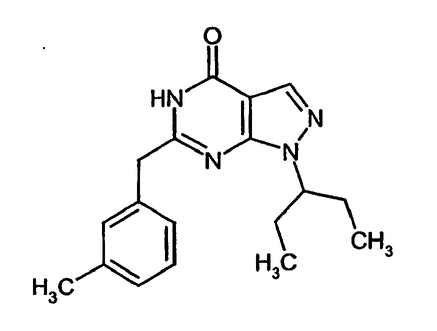

Analog zu Beispiel 1 wird das Produkt ausgehend von 200 mg (1.0 mmol) 5-Amino-1-(1-ethylpropyl)-1H-pyrazol-4-carboxamid und 534 mg (3.0 mmol) (3-Methylphenyl)essigsäureethylester erhalten.
Ausbeute: 187 mg (60 % d.Th.)
MS (ESI): m/z = 311 (M+H)⁺
Smp.: 128°C
¹H-NMR (200 MHz, DMSO-d₆): δ = 12.25 (s, 1H), 8.0 (s, 1H), 7.25-7.0 (m, 4H), 4.5 (m, 1H), 3.95 (s, 2H), 2.25 (s, 3H), 2.0-1.7 (m, 4H), 0.6 (t, 6H, *J* = 7.5 Hz) ppm.

### Beispiel 11

### 1-(1-Ethylpropyl)-6-[3-(trifluormethyl)benzyl]-1,5-dihydro-4H-pyrazolo[3,4-d]pyrimidin-4-on

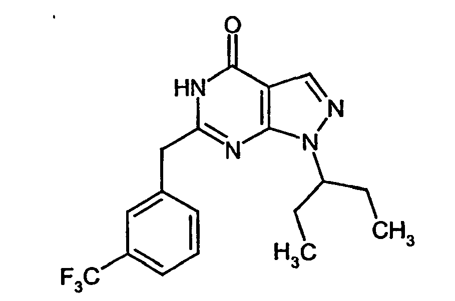

Analog zu Beispiel 1 wird das Produkt ausgehend von 150 mg (0.75 mmol) 5-Amino-1-(1-ethylpropyl)-1H-pyrazol-4-carboxamid und 490 mg (2.25 mmol) (3-Trifluormethylphenyl)essigsäuremethylester erhalten.
Ausbeute: 159 mg (58 % d.Th.)
MS (ESI): m/z = 365 (M+H)⁺
Smp.: 120°C
¹H-NMR (400 MHz, DMSO-d₆): δ = 12.3 (s, 1H), 8.0 (s, 1H), 7.7 (s, 1H), 7.7-7.5 (m, 3H), 4.4 (m, 1H), 4.1 (s, 2H), 1.95-1.75 (m, 4H), 0.6 (t, 6H, J= 7.5 Hz) ppm.

### Beispiel 12

### 1-Cyclopentyl-6-(3-nitrobenzyl)-1,5-dihydro-4H-pyrazolo [3,4-d]pyrimidin-4-on

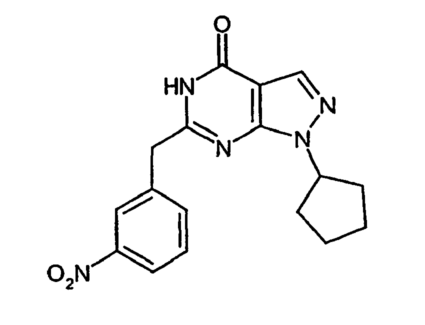

Analog zu Beispiel 1 wird das Produkt ausgehend von 668 mg (3.44 mmol) 5-Amino-1-cyclopentyl-1H-pyrazol-4-carboxamid und 3.5 g (13.7 mmol) 3-Nitrophenylessigsäureethylester erhalten.
Ausbeute: 10 mg (1 % d.Th.)
MS (ESI): m/z = 340 (M+H)⁺
¹H-NMR (300 MHz, DMSO-d₆): δ = 12.3 (s, 1H), 8.3 (s, 1H), 8.15 (m, 1H), 8.0 (s, 1H), 7.8 (d, 1H, *J* = 8 Hz), 7.6 (t, 1H, *J* = 8 Hz), 5.0 (m, 1H), 4.15 (s, 2H), 2.1-1.6 (m, 8H).

### Beispiel 13

### 6-(3-Chlorbenzyl)-1-cyclopentyl-1,5-dihydro-4H-pyrazolo[3,4-d]pyrimidin-4-thion

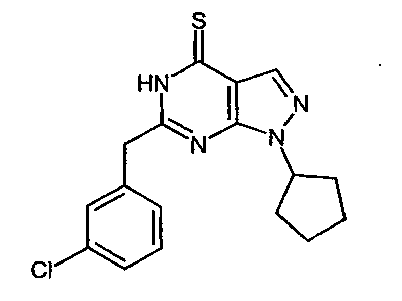

Eine Lösung von 50 mg (0.15 mmol) 6-(3-Chlorbenzyl)-1-cyclopentyl-1,5-dihydro-4H-pyrazolo[3,4-d]pyrimidin-4-on (Beispiel 1) in 1 ml Pyridin wird bei Raumtemperatur mit 50 mg (0.23 mmol, 1.5 equiv.) Diphosphorpentasulfid versetzt und anschließend über Nacht unter Rückfluß gerührt. Nach dem Abkühlen wird die Reaktionslösung mit 10 ml eiskalter 2.5%-iger Natriumhydrogencarbonat-Lösung versetzt und dreimal mit Essigsäureethylester extrahiert. Die vereinigten organischen Phasen werden mit gesättigter Kochsalzlösung gewaschen, über Natriumsulfat getrocknet und im Vakuum eingeengt. Das Rohprodukt wird über präparative HPLC gereinigt.
Ausbeute: 36 mg (68% d.Th.)
MS (ESI): m/z = 345 (M+H)⁺
Smp.: 154°C
¹H-NMR (300 MHz, DMSO-d₆): δ = 13.6 (s, 1H), 8.15 (s, 1H), 7.5 (s, 1H), 7.4-7.25 (m, 3H), 5.05 (m, 1H), 4.1 (s, 2H), 2.1-1.6 (m, 8H).

### Beispiel 14

### 1-Cyclopentyl-6-[2-(trifluormethoxy)benzyl]-1,5-dihydro-4H-pyrazolo[3,4-d]-pyrimidin-4-on

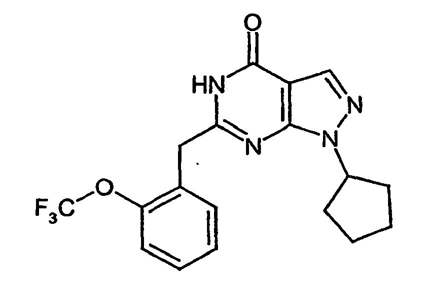

Analog zu Beispiel 1 wird das Produkt ausgehend von 50 mg (0.26 mmol) 5-Amino-1-cyclopentyl-1H-pyrazol-4-carboxamid und 301 mg (1.29 mmol) [2-(Trifluormethoxy)phenyl]essigsäuremethylester erhalten.
Ausbeute: 64 mg (63 % d.Th.)
MS (DCI): m/z = 379 (M+H)⁺
Smp.: 161°C
¹H-NMR (400 MHz, DMSO-d₆): δ = 12.25 (s, 1H), 8.0 (s, 1H), 7.5-7.3 (m, 4H), 4.9 (m, 1H), 4.1 (s, 2H), 2.05-1.5 (m, 8H) ppm.

## Patentansprüche

1. Verbindungen der Formel 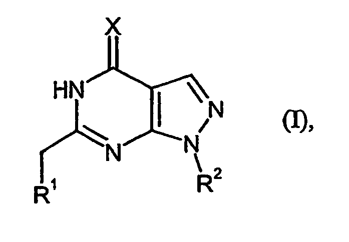 in welcher
R¹ Phenyl, welches mit 1 bis 5 Substituenten unabhängig voneinander ausgewählt aus der Gruppe Halogen, C₁-C₆-Alkyl, Trifluormethyl, Trifluormethoxy, Cyano, Hydroxy, Nitro und C₁-C₆-Alkoxy substituert ist,
R² Pentan-3-yl, C₄-C₆-Cycloalkyl,
X Sauerstoff oder Schwefel,
bedeuten,
sowie deren Salze, Solvate und/oder Solvate der Salze.

2. Verbindungen nach Anspruch 1, wobei
R¹ Phenyl, welches mit 1 bis 3 Substituenten unabhängig voneinander ausgewählt aus der Gruppe Fluor, Chlor, Brom, C₁-C₄-Alkyl, Trifluormethyl, Trifluormethoxy, Cyano, Hydroxy, Nitro und C₁-C₄-Alkoxy substituert ist,
R² Pentan-3-yl, C₅-C₆-Cycloalkyl,
X Sauerstoff oder Schwefel,
bedeuten,
sowie deren Salze, Solvate und/oder Solvate der Salze.

3. Verbindungen nach Ansprüchen 1 und 2 der Formel 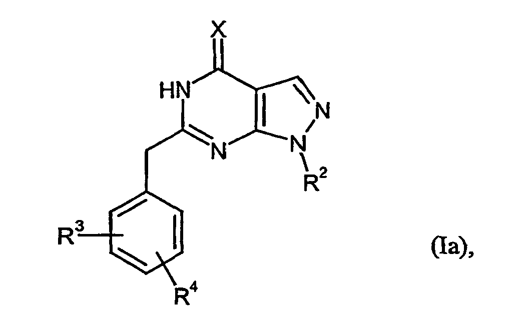 in welcher
R³ Wasserstoff oder Chlor,
R⁴ Fluor, Chlor, Brom, Methyl, Trifluormethyl,
R² Pentan-3-yl, Cyclopentyl,
X Sauerstoff oder Schwefel,
bedeuten,
sowie deren Salze, Solvate und/oder Solvate der Salze.

4. Verbindungen nach Ansprüchen 1 bis 3 der Formel (Ia), wobei
R³ Wasserstoff oder Chlor,
R⁴ Fluor, Chlor, Brom, Methyl, Trifluormethyl,
R² Pentan-3-yl, Cyclopentyl,
X Sauerstoff,
bedeuten,
sowie deren Salze, Solvate und/oder Solvate der Salze.

5. Verfahren zur Herstellung von Verbindungen nach Anspruch 1, **dadurch gekennzeichnet, dass** man
[A] Verbindungen der Formel 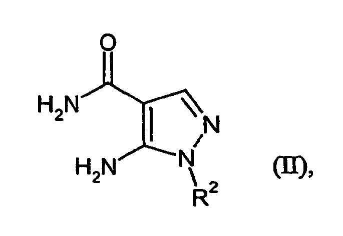 in welcher
R² die in Anspruch 1 angegebenen Bedeutungen aufweist,
durch Umsetzung mit einer Verbindung der Formel
R¹-CH₂C(O)-Z (IIIa),
in welcher
R¹ die in Anspruch 1 angegebenen Bedeutungen aufweist,
und
Z für Chlor oder Brom steht,
zunächst in Gegenwart einer Base in Verbindungen der Formel 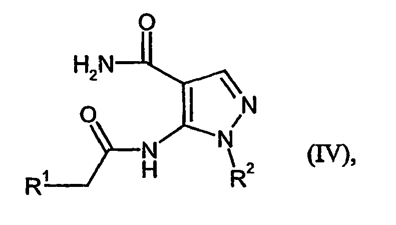 in welcher
R¹ und R² die in Anspruch 1 angegebenen Bedeutungen aufweisen,
überführt, dann in Gegenwart einer Base zu Verbindungen der Formel 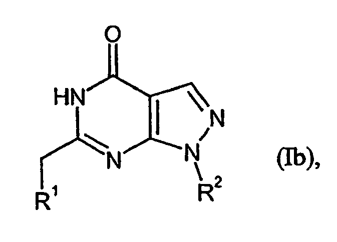 in welcher
R¹ und R² die in Anspruch 1 angegebenen Bedeutungen aufweisen,
cyclisiert,
oder
[B] Verbindungen der Formel (II) unter direkter Cyclisierung zu (Ib) mit einer Verbindung der Formel
R¹-CH₂-C(O)-OR⁵ (IIIb),
in welcher
R¹ die in Anspruch 1 angegebenen Bedeutungen aufweist,
und
R⁵ für Methyl oder Ethyl steht,
in Gegenwart einer Base umsetzt,
oder
[C] Verbindungen der Formel 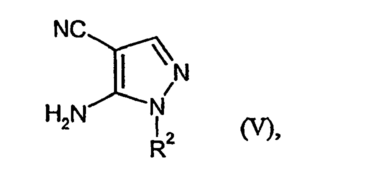 in welcher
R² die in Anspruch 1 angegebenen Bedeutungen aufweist,
zunächst durch Umsetzung mit einer Verbindung der Formel (IIIa) in Gegenwart einer Base in Verbindungen der Formel 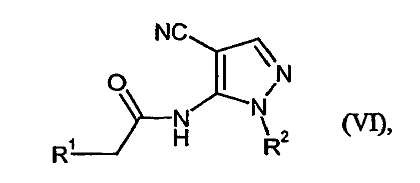 in welcher
R¹ und R² die in Anspruch 1 angegebenen Bedeutungen aufweisen,
überführt,
und diese in einem zweiten Schritt in Gegenwart einer Base und eines Oxidationsmittels zu (Ib) cyclisiert,
und die Verbindungen der Formel (Ib) gegebenenfalls dann durch Umsetzung mit einem Schwefelungsmittel wie beispielsweise Diphosphorpentasulfid in die Thiono-Derivate der Formel 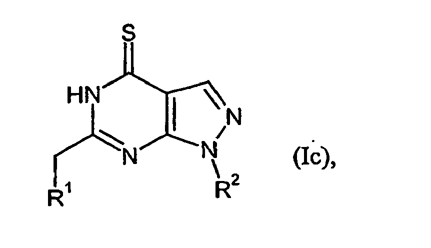 in welcher
R¹ und R² die in Anspruch 1 angegebenen Bedeutungen aufweisen,
überführt,
und die resultierenden Verbindungen der Formel (I) gegebenenfalls mit den entsprechenden (i) Lösungsmitteln und/oder (ii) Basen oder Säuren zu ihren Solvaten, Salzen und/oder Solvaten der Salze umsetzt.

6. Verbindungen nach einem der Ansprüche 1 bis 4 zur Behandlung und/oder Prophylaxe von Krankheiten.

7. Arzneimittel enthaltend mindestens eine der Verbindungen nach einem der Ansprüche 1 bis 4 und mindestens einen pharmazeutisch verträglichen, im wesentlichen nichtgiftigen Träger oder Exzipienten.

8. Verwendung der Verbindungen nach einem der Ansprüche 1 bis 4 zur Herstellung eines Arzneimittels zur Prophylaxe und/oder Behandlung von Störungen der Wahrnehmung, Konzentrationsleistung, Lern- und/oder Gedächtnisleistung.

9. Verwendung nach Anspruch 8, wobei die Störung eine Folge der Alzheimer'schen Krankheit ist.

10. Verwendung der Verbindungen nach einem der Ansprüche 1 bis 4 zur Herstellung eines Arzneimittels zur Verbesserung der Wahrnehmung, Konzentrationsleistung, Lern- und/oder Gedächtnisleistung.

## Claims

1. Compounds of the formula 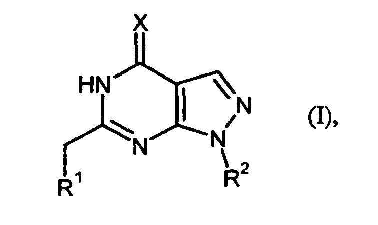 in which
R¹ is phenyl which is substituted by 1 to 5 substituents independently of one another selected from the group of halogen, C₁-C₆-alkyl, trifluoromethyl, trifluoromethoxy, cyano, hydroxy, nitro and C₁-C₆₋alkoxy,
R² is pentan-3-yl, C₄-C₆-cycloalkyl,
X is oxygen or sulphfur,
and the salts, solvates and/or solvates of the salts thereof.

2. Compounds according to Claim 1, where
R¹ is phenyl which is substituted by 1 to 3 substituents independently of one another selected from the group of fluorine, chlorine, bromine, C₁-C₄-alkyl, trifluoromethyl, trifluoromethoxy, cyano, hydroxy, nitro and C₁-C₄-alkoxy,
R² is pentan-3-yl, C₅-C₆-cycloalkyl,
X is oxygen or sulphur,
and the salts, solvates and/or solvates of the salts thereof.

3. Compounds according to Claims 1 and 2 of the formula 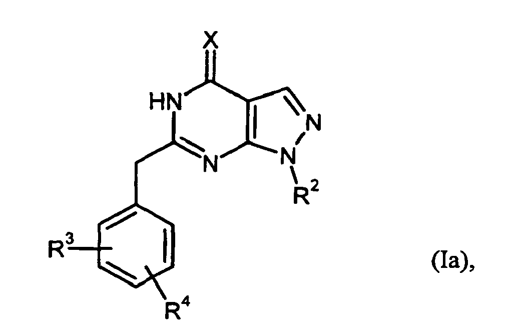 in which
R³ is hydrogen or chlorine,
R⁴ is fluorine, chlorine, bromine, methyl, trifluoromethyl,
R² is pentan-3-yl, cyclopentyl,
X is oxygen or sulphur,
and the salts, solvates and/or solvates of the salts thereof.

4. Compounds according to Claims 1 to 3 of the formula (Ia), where
R³ is hydrogen or chlorine,
R⁴ is fluorine, chlorine, bromine, methyl, trifluoromethyl,
R² is pentan-3-yl, cyclopentyl,
X is oxygen,
and the salts, solvates and/or solvates of the salts thereof.

5. Process for preparing compounds according to Claim 1, **characterized in that**
[A] compounds of the formula 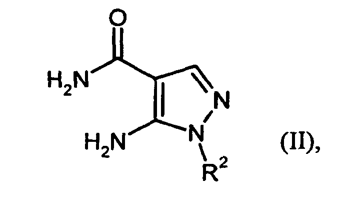 in which
R² has the meanings indicated in Claim 1,
are converted by reaction with a compound of the formula
R¹-CH₂-C(O)-Z (IIIa),
in which
R¹ has the meanings indicated in Claim 1,
and
Z is chlorine or bromine,
initially in the presence of a base into compounds of the formula 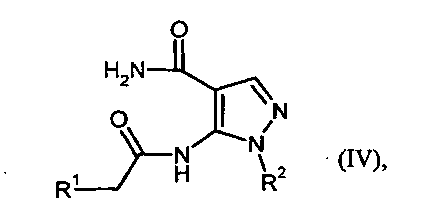 in which
R¹ and R² have the meanings indicated in Claim 1,
then cyclized in the presence of a base to compounds of the formula 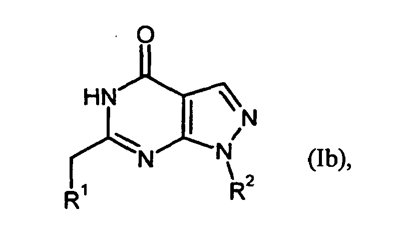 in which
R¹ and R² have the meanings indicated in Claim 1,
or
[B] compounds of the formula (II) are reacted with direct cyclization to (Ib) with a compound of the formula
R¹-CH₂-C(O)-OR⁵ (IIIb),
in which
R¹ has the meanings indicated in Claim 1,
and
R⁵ is methyl or ethyl,
in the presence of a base,
or
[C] compounds of the formula 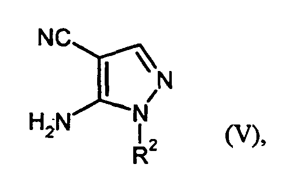
in which
R² has the meanings indicated in Claim 1,
are converted initially by reaction with a compound of the formula (IIIa) in the presence of a base into compounds of the formula 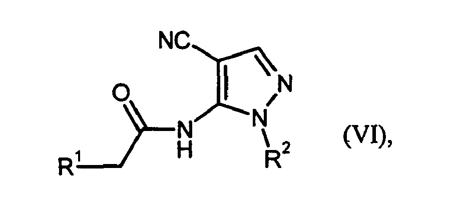 in which
R¹ and R² have the meanings indicated in Claim 1,
and the latter are cyclized in a second step in the presence of a base and of an oxidizing agent to (Ib),
and the compounds of the formula (Ib) are then converted where appropriate by reaction with a sulphurizing agent such as, for example, diphosphorus pentasulphide into the thiono derivatives of the formula 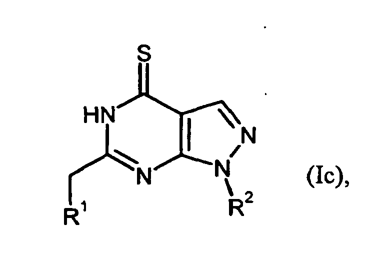 in which
R¹ and R² have the meanings indicated in Claim 1,
and the resulting compounds of the formula (I) are reacted where appropriate with the appropriate (i) solvents and/or (ii) bases or acids to give the solvates, salts and/or solvates of the salts thereof.

6. Compounds according to any of Claims 1 to 4 for the treatment and/or prophylaxis of diseases.

7. Medicament comprising at least one of the compounds according to any of Claims 1 to 4 and at least one pharmaceutically acceptable, essentially nontoxic carrier or excipient.

8. Use of the compounds according to any of Claims 1 to 4 for producing a medicament for the prophylaxis and/or treatment of impairments of perception, concentration, learning and/or memory.

9. Use according to Claim 8, where the impairment is a consequence of Alzheimer's disease.

10. Use of the compounds according to any of Claims 1 to 4 for producing a medicament for improving perception, concentration, learning and/or memory.

## Revendications

1. Composés de formule 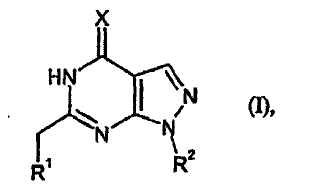 dans laquelle
R¹ représente un reste phényle, qui porte 1 à 5 substituants choisis, indépendamment les uns des autres, dans le groupe des substituants halogéno, alkyle en C₁ à C₆, trifluorométhyle, trifluorométhoxy, cyano, hydroxy, nitro et alkoxy en C₁ à C₆,
R² est un reste pentane-3-yle, cycloalkyle en C₄ à C₆,
X représente l'oxygène ou le soufre,
ainsi que leurs sels, leurs produits de solvatation et/ou les produits de solvatation des sels.

2. Composés suivant la revendication 1, dans lesquels
R¹ représente un reste phényle, qui porte 1 à 3 substituants choisis, indépendamment les uns des autres, dans le groupe des substituants fluoro, chloro, bromo, alkyle en C₁ à C₄, trifluorométhyle, trifluorométhoxy, cyano, hydroxy, nitro et alkoxy en C₁ à C₄,
R² est un reste pentane-3-yle, cycloalkyle en C₅ ou C₆,
X représente l'oxygène ou le soufre,
ainsi que leurs sels, leurs produits de solvatation et/ou les produits de solvatation des sels.

3. Composés suivant les revendications 1 et 2, de formule 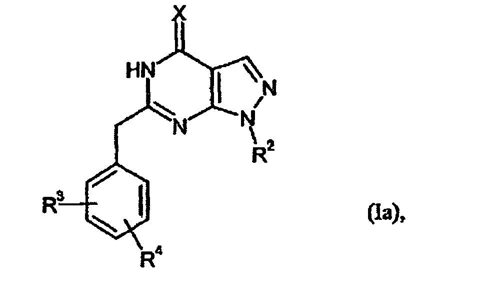 dans laquelle
R³ représente l'oxygène ou le chlore,
R⁴ représente le fluor, le chlore, le brome, un reste méthyle, trifluorométhyle,
R² est un reste pentane-3-yle, cyclopentyle,
X représente l'oxygène ou le soufre,
ainsi que leurs sels, leurs produits de solvatation et/ou les produits de solvatation des sels.

4. Composés suivant les revendications 1 à 3, de formule (Ia) dans laquelle
R³ représente l'hydrogène ou le chlore,
R⁴ représente le fluor, le chlore, le brome, un reste méthyle, trifluorométhyle,
R² est un reste pentane-3-yle, cyclopentyle,
X représente l'oxygène,
ainsi que leurs sels, leurs produits de solvatation et/ou les produits de solvatation des sels.

5. Procédé de production de composés suivant la revendication 1, **caractérisé en ce que**
[A] on transforme tout d'abord des composés de formule 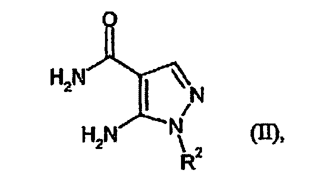 dans laquelle
R² a les définitions indiquées dans la revendication 1, par réaction avec un composé de formule
R¹-CH₂-C(O)-Z (IIIa)
dans laquelle
R¹ a les définitions indiquées dans la revendication 1,
et
Z représente le chlore ou le brome,
en présence d'une base, en composés de formule 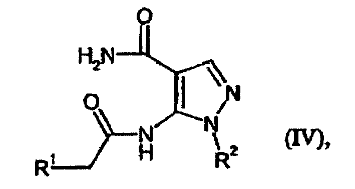 dans laquelle
R¹ et R² ont les définitions indiquées dans la revendication 1,
qu'on cyclise ensuite en présence d'une base en composés de formule 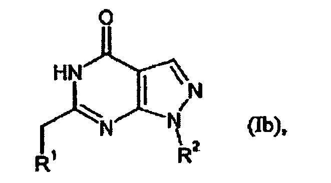 dans laquelle
R¹ et R² ont les définitions indiquées dans la revendication 1,
ou bien
[B] on fait réagir des composés de formule (II) avec cyclisation directe en (Ib) avec un composé de formule
R¹-CH₂-C(O)-OR⁵ (IIIb)
dans laquelle
R¹ a les définitions indiquées dans la revendication 1,
et
R⁵ est un reste méthyle ou éthyle, en présence d'une base
ou bien
[C] on transforme tout d'abord des composés de formule 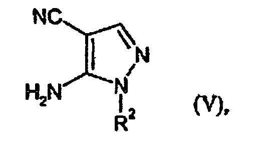
dans laquelle
R² a les définitions indiquées dans la revendication 1, par réaction avec un composé de formule (IIIa) en présence d'une base, en composés de formule 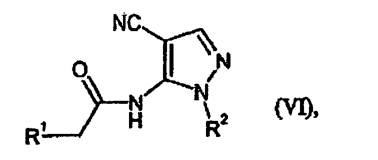 dans laquelle
R¹ et R² ont les définitions indiquées dans la revendication 1,
qu'on cyclise dans une seconde étape en (Ib) en présence d'une base et d'un agent oxydant,
et on transforme ensuite le cas échéant les composés de formule (Ib) par réaction avec un agent de sulfuration tel que, par exemple, le pentasulfure de diphosphore en les dérivés thiono de formule 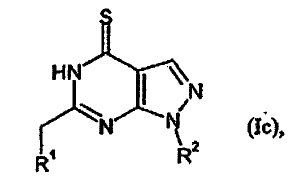 dans laquelle
R¹ et R² ont les définitions indiquées dans la revendication 1,
et on fait éventuellement réagir les composés résultant de formule (I) avec (i) les solvants correspondants et/ou (ii) les bases ou les acides correspondants pour former leurs produits de solvatation, leurs sels et/ou les produits de solvatation des sels.

6. Composés suivant l'une des revendications 1 à 4, destinés au traitement et/ou à la prophylaxie de maladies.

7. Médicament contenant au moins l'un des composés suivant l'une des revendications 1 à 4 et au moins un support ou excipient essentiellement non toxique, acceptable du point de vue pharmaceutique.

8. Utilisation des composés suivant l'une des revendications 1 à 4 pour la fabrication d'un médicament destiné à la prophylaxie et/ou au traitement de troubles de la perception, de la capacité de concentration, de la capacité d'apprentissage et/ou de mémoire.

9. Utilisation suivant la revendication 8, le trouble étant une conséquence de la maladie d'Alzheimer.

10. Utilisation des composés suivant l'une des revendications 1 à 4 pour la fabrication d'un médicament destiné à améliorer la perception, la capacité de concentration, la capacité d'apprentissage et/ou de mémoire.
